(19) Europäisches Patentamt European Patent Office Office européen des brevets

(11) **EP 3 473 202 B1**

(12) **EUROPEAN PATENT SPECIFICATION**

(45) Date of publication and mention
of the grant of the patent:
**03.03.2021 Bulletin 2021/09**

(51) Int Cl.:
***B25J 13/02*** *(2006.01)*        ***A61B 34/10*** *(2016.01)*
***A61B 34/30*** *(2016.01)*        *A61B 34/00* *(2016.01)*
***A61B 90/00*** *(2016.01)*

(21) Application number: **17814810.2**

(22) Date of filing: **22.06.2017**

(86) International application number:
**PCT/ES2017/070456**

(87) International publication number:
**WO 2017/220844 (28.12.2017 Gazette 2017/52)**

(54) **ROBOTIC SYSTEM FOR MINIMALLY INVASIVE SURGERY**

ROBOTERSYSTEM FÜR MINIMAL INVASIVE CHIRURGIE

SYSTÈME ROBOTIQUE POUR CHIRURGIE MINI-INVASIVE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.06.2016 ES 201630855**

(43) Date of publication of application:
**24.04.2019 Bulletin 2019/17**

(73) Proprietor: **Universidad De Malaga
29071 Málaga (ES)**

(72) Inventors:
• **BAUZANO NÚÑEZ, Enrique**
**29590 Málaga (ES)**
• **LÓPEZ CASADO, Mª Carmen**
**29590 Málaga (ES)**
• **MUÑOZ MARTÍNEZ, Víctor Fernando**
**29590 Málaga (ES)**
• **PÉREZ DEL PULGAR MANCEBO, Carlos Jesús**
**29590 Málaga (ES)**

(74) Representative: **Balder IP Law, S.L.
Paseo de la Castellana 93
5ª planta
28046 Madrid (ES)**

(56) References cited:
**EP-A1- 1 915 963        WO-A1-2005/039835
WO-A1-2015/158756        WO-A1-2016/064632
WO-A2-2007/136768        US-A1- 2013 012 930**

• **AHAIYANG JIN et al.: "DESIGN AND CONTROL
STRATEGYOF ROBOTIC SPINAL SURGICAL
SYSTEM", Proceedings of the 2011 IEEE /ICME
May 22 - 25, 25 May 2011 (2011-05-25),
XP031993657, Harbin, China [retrieved on
2017-11-15]**

EP 3 473 202 B1

**EP 3 473 202 B1**

## Description

## Technical field

The present invention belongs to the fields of surgery and robotics, specifically to the field of surgical support systems, and more precisely to robotic systems specially designed as surgical instruments.

## Prior art

Minimally invasive surgery consists of performing an intervention through a minimum number of small incisions in the patient, about 1-2 cm in length. Specifically, laparoscopic surgery is a type of minimally invasive surgery in which tools in the form of a long shaft are used to perform the surgical intervention. The number of tools depends on the intervention, but at least one of them must be able to transmit to the surgeon an image of the surgical area, and it normally consists of an optic with a camera coupled to the end outside the patient. This surgical technique can be used in a number of interventions, such as abdominal (cholecystectomy, nephrectomy, prostatectomy...), intracranial (tumour resection...) or trauma (arthroscopy, orthopaedic...) interventions. In the case of abdominal laparoscopic surgery, the creation of an abdominal vault by means of inserting an inert gas (normally carbon dioxide) is required for allowing mobility of the laparoscopic tools, such that the gas is occluded in the abdominal cavity by using special valves called trocars, which are placed in the abdominal incisions and allow passage of the surgical tools.

Laparoscopic surgery has a series of advantages for the patient, such as the presence of smaller scars after the intervention and a much shorter postoperative recovery time compared to open surgery. However, laparoscopic surgery limits the skills of the surgeon relative to the skills for an open intervention. Some examples are the loss of three-dimensional vision upon displaying an image on a screen, the inversion of movements of the laparoscopic tools resulting from the restricted movement inherent to the point of insertion, which is located at the incision or fulcrum point, as well as the loss of tactile sensation given that the fingers of the surgeon are not in direct contact with the patient.

A solution to these problems proposed by the state of the art consists of using a robotic device as an intermediate tool for the surgeon. These robotic devices can have one or several manipulator arms according to the number of tools they are able to manipulate, and can be classified in two main groups: robotic assistants and teleoperated robots. Robotic assistants are able to perform specific tasks in the surgical area in an autonomous manner or through simple commands controlled by the surgeon through a control interface. In contrast, in teleoperated robots the movements of the surgical robot or slave system directly correspond to the movements performed by the surgeon (normally the hands) or master system, to thus improve the skills and precision of the surgeon with the laparoscopic tools, such that the robot almost or completely lacks autonomous movement capabilities.

Robotic assistants have the advantage that they do not require direct human intervention except to control the commands desired by the surgeon, such that they behave like a human assistant to the surgeon for the purposes of the intervention. The biggest drawback is that a robotic assistant is normally programmed to perform very specific tasks, so it cannot be used in a catch-all manner during an intervention like a human assistant. For example, Spanish patent ES2298051B2 describes a robotic assistant capable of handling the laparoscopic camera by means of voice commands given by the surgeon, with other characteristics such as flexibility in the positioning around the patient as a result of a wheel-based drive system of the structure and the complete absence of cables since it can work with batteries.

As regards the group of teleoperated robots, as mentioned above, they seek to improve the skills of the surgeon. To that end, the surgical robot (slave system) replaces the main surgeon in the area around the patient, such that the new location of the surgeon will consist of a platform referred to as the console (master system) where the surgeon will have available all the tools required to control the surgical robot and carry out the intervention.

Various skills of the surgeon can be improved from the console by means of the use of specific devices. From the vantage point of vision, patent application US20070276423A1 proposes the use of a three-dimensional vision system of by means using a stereoscopic optic, which transmits each of the images to the corresponding eye by means of mirrors.

The movement of the manipulator arm/arms making up the surgical robot is controlled by the surgeon through special mechanical devices with positioning sensors called haptic devices or haptics, which are located in the console and are usually handled by the hands of the surgeon. As specified in patent US7025064B2, these haptic devices allow not only handling the surgical robot, but they can also improve laparoscopic tool movement precision by means of applying scale factors which reduce movement of the laparoscopic tools relative to the movement of the hands of the surgeon. The use of haptic devices is not the only means whereby the surgeon can direct the movement of laparoscopic tools, for example international patent application WO2011125007A1 proposes the use of an eye tracking system capable of guiding the laparoscopic camera according to the direction of the surgeon's gaze.

Haptic devices can also include servo actuator elements for applying reaction forces on the surgeon, such that the surgeon can perceive the sensation of the laparoscopic tools being handled in contact with the internal tissue of the patient. To that end, the surgical robot must have at least one force and/or moment measurement device for each manipulator arm whereby the contact pressures between the laparoscopic tools and the internal tissue of the patient or additional surgical instruments can be translated. For example, patent application US2013012930A1 proposes sending these measurements in the form of an electrical signal to the servo actuators coupled in haptic devices by means of a communication control system which must comply with a series of specific requirements so as to ensure the stability of the interaction between the slave system (surgical robot) and master system (console), known as haptic force feedback. This feedback on the forces of interaction between the surgical robot and the internal tissue of the patient does not have to be about pressure alone, rather there are other proposals, such as that of patent application US2014005682A1, which allow feedback on tactile sensations, such as the roughness of a surface, by means of using ultrasonic surgical tools.

One of the main problems with the feedback of haptic forces in a surgical robot not considered in US2013012930A1 or in US2014005682A1 resides in the fact that there are normally two areas of contact between the laparoscopic tool handled by the surgical robot and the patient: the abdominal area on which the incision is made (hereinafter fulcrum point) and the area of internal tissue to be manipulated by the surgeon. As a result, for a correct tactile perception of the surgical area, the contribution of both interactions in the measurement of the force and/or moment measurement device must be separated, as disclosed for example in US2013/0012930A1.

The contact force between the laparoscopic tool and the fulcrum point largely depends on the type of mechanism performing the orientation movements of the laparoscopic tool handled by the surgical robot. For example, in the aforementioned documents US7025064B2 and ES2298051B2, a passive actuation mechanism that limits the force exerted by the surgical tool on the abdominal skin but increases positioning uncertainty resulting from play with the trocar is used. In the aforementioned US20070276423A1, a mechanism called a remote centre of rotation is used, which mechanically transfers the centre of rotation of the surgical tool to the fulcrum point; therefore, the problem with this method can be reduced to initially calibrating the position of the remote centre of rotation, such that if said centre changes during the intervention it will have to be recalibrated. Patent application US2015359597A1 proposes the use of a second manipulator arm to know the location of the fulcrum point, such that this second manipulator holds the surgical tool at the point of insertion of the laparoscopic tool in the patient through its end effector. Further background prior art is disclosed in WO 2015/158756 and WO 2016/064632.

In summary, the state of the art has the following limitations: On one hand, there are proposals which allow the surgeon to perceive the interaction of forces between the surgical tools and the patient, but such proposals do not take into account the overlapping of forces exerted by the laparoscopic tool on the point of insertion and the manipulation forces relative to the internal tissue of the patient. On the other hand, the proposals for the control of movements of laparoscopic tools handled by a surgical robot depend on mechanisms which either produce certain play/imprecision in the positioning of surgical tools, or else require offline recalibration, both at the beginning of the intervention and in the case of movement of the fulcrum point resulting from factors such as a possible movement of the patient on the operating table. Finally, the proposed surgical robots described only allow one type of correspondence between the movement of the haptic devices and of the laparoscopic tools, such that the position of the end effector of the haptic device can only correspond with the position of the distal end of the laparoscopic tool handled by the surgical robot.

Description of the invention

The invention is set out in the appended claims. Further embodiments are given in the dependent claims. The description also refers to a method, which is to be understood merely as an example suitable for understanding the invention, the method as such being excluded from the claimed subject-matter. A remotely operated surgical robot overcoming the drawbacks identified in conventional methods for controlling surgical robots is provided.

In particular, at least one robotic arm is controlled such that a surgical tool coupled to the robotic arm is moved or oriented correctly within the cavity defined by the incision made in the patient. Furthermore, the forces applied by the surgical tool are fed to a control unit or console to provide sensory information to the surgeon who is remotely manipulating the surgical tool, thus helping the surgeon move the tool as if he or she were directly manipulating the patient.

A robotic system for minimally invasive surgery is provided, wherein the robotic system comprises: a control console comprising at least one actuating device and one haptic device which in turn comprises one or more positioning sensors and servo actuators; and at least one robotic unit comprising: a manipulator arm, an effector arranged at the distal end of said manipulator arm, said effector being equipped with at least one force and moment sensor, at least one actuator, and a minimally invasive instrument coupled to said effector, wherein the distal end of said minimally invasive instrument is configured for being introduced into a cavity of the body of a patient through a fulcrum point. The system is configured to carry out the steps of: programming a position and orientation for the effector based on a relative movement of the haptic device, reference effector coordinates, effector coordinates according to a model of the robotic unit, and an estimated position of the fulcrum; based on the effector coordinates according to the model of the robotic unit and the programmed position and orientation of the effector, obtaining articular speeds and positions required so that each degree of freedom of the robotic unit moved by the actuator, together, makes it possible to reach the next programmed position and orientation of the effector; moving the effector by means of the at least one actuator according to said articular speeds and positions; measuring by means of the at least one force and moment sensor coupled to the effector forces and moments exerted by the effector and by said minimally invasive instrument coupled thereto when this movement is performed; determining what percentage of the force and moment measurement is contributed as a result of the interaction with the fulcrum point or the interaction with the internal tissue of the patient; wherein this determination is made based on the magnitude of said measurement by applying a parameterised sigmoid function for each contribution; estimating the position of the fulcrum again, where that estimation is performed based on the contribution resulting from the interaction with the fulcrum point and on the coordinates of the effector according to the model of the robotic unit; estimating the stiffness of the tissue in contact with the distal end of the minimally invasive instrument and calculating a simulated reaction force; sending this simulated reaction force to at least one servo actuator of the haptic device for it to be provided to the hand of the surgeon.

In a possible embodiment, the coordinates of the effector according to the model of the robotic unit are obtained based on an articular position of the model.

In a possible embodiment, the relative movement of the haptic device is obtained based on the difference between an absolute position and torsion of the haptic device and a reference position and torsion. In a more particular embodiment, scaling factors of position $K_P$ and torsion $K_y$ established by the surgeon to increase movement precision, and a factor of reduction $K_s$ related to the simulated reaction force are applied to that difference between an absolute position and torsion of the haptic device and a reference position and torsion.

In a possible embodiment, to estimate the fulcrum, the external distance p along the axis of the minimally invasive instrument at which the fulcrum point is located relative to the position of the effector according to that model of the robotic unit is estimated.

In a possible embodiment, the estimation of the stiffness of the tissue in contact with the distal end of the minimally invasive instrument and calculation of a simulated reaction force are performed based on the contribution resulting from the interaction with the internal tissue of the patient, the effector coordinates according to the model of the robotic unit, and the movement of the haptic device performed by the hand of the surgeon.

In a possible embodiment, the calculation of a simulated reaction force is performed based on the following expression:

$$^{\{H\}}\boldsymbol{F_H} = -K_F K_T (^{\{H\}}\boldsymbol{P_H} - {}^{\{H\}}\boldsymbol{P_H^0})$$

where $^{\{H\}}\mathbf{F_H}$ represents the simulated force, $K_F$ is a scaling factor, with $K_F < 1$, $K_T$ is the dynamic stiffness, $^{\{H\}}\mathbf{P_H}$ is the current haptic position and $^{\{H\}}\mathbf{P_H^0}$ is the last haptic position.

In a possible embodiment, the system is further configured to: based on the position and orientation of the effector according to the model of the robotic unit, the programmed position and orientation and the position and orientation of other robotic units, if any, verifying that the programmed position and orientation for the final effector of the robotic unit comply with safety criteria, and restricting said position and orientation should they fail to comply with said criteria.

In a possible embodiment, the system is further configured to: through an interface of the control console, selecting a type of movement of the minimally invasive instrument: a first type in which the movement of the haptic device is related to the movement of the distal end of the minimally invasive instrument; or a second type in which the movement of the haptic device is related to the movement of the effector of the manipulator arm, such that the pivoting movements of a manual minimally invasive instrument are simulated through the haptic device.

In a possible embodiment, any of the preceding steps is carried out if said actuating device is pushed or actuated.

In a preferred embodiment, the actuating device is a pedal.

According to another aspect of the invention, a computer software product comprising computer program code/instructions is provided to cause the robotic system to perform the steps described above.

According to a final aspect of the invention, computer-readable support/storage media that stores program code / instructions for performing the method/steps above when executed on the robotic system is provided.

The method is implemented in a system which allows the remote manipulation of laparoscopic tools manipulated by a surgical robot (slave system) through an interface or console (master system) handled by a human user. The master system includes at least one screen which allows displaying the surgical area in two or three dimensions in real time, an actuating device for activating or deactivating the handling of the laparoscopic tools and two servo actuated and sensorised mechanical devices (haptics which, on one hand, record the movements performed by the hands of the user for moving and orienting respective reference points determined by each haptic, and on the other hand enable a force to be transmitted to the hands of the user through the servo actuators in the hands of the user. The slave system can be made up of one or several modules, each of which comprises independent servo actuated and sensorised devices (robots/manipulator arms), at the distal ends of which there is coupled a laparoscopic tool, and the function of which is based on reproducing the movements recorded by its associated haptic device for said laparoscopic tool to move synchronously with the hand of the user. The haptic device and the manipulator arm can be separated from one another a certain distance and communicate through the transmission of electrical signals through a communications cable or wirelessly.

The relation existing between the movements of the hand of the user (haptic) and the movements of the laparoscopic tool (manipulator arm) can be established in two ways, to be selected by the user. The first one is based on defining a translation/orientation of the haptic device as a translation/orientation of the distal end of the laparoscopic tool (for example, clamp), such that a Cartesian movement of the hand of the user corresponds to a Cartesian movement of the distal end of the laparoscopic tool. The second one translates the translation/orientation of the haptic device into an equivalent translation/orientation of the proximal end of the laparoscopic tool, that is, the position/orientation of the end of the laparoscopic tool which is coupled to the manipulator. In turn, the system allows a completely adjustable scaling of movements, such that a movement of 1 cm in the haptic device, for example, is translated into a movement of 1 mm in the manipulator device (1:10 scale).

The pivoting movements of the laparoscopic tool are performed around the fulcrum, which introduces a ligature (two Cartesian degrees of freedom are lost in the movement) which prevents the free movement of the laparoscopic tool handled by the manipulator. As a result, the manipulation system has an element which, in a transparent manner, geometrically translates the movements commanded by the user into pivoting movements of the laparoscopic tool, the centre of which is located at the fulcrum.

Each manipulator arm of each surgical robot has a force and moment measurement device coupled to its end effector which enables a measurement of the contact forces and moments between the laparoscopic tool and the patient to be obtained, which can be generated in two well defined areas: the fulcrum and the distal end. Contact forces on the fulcrum are called reaction forces and occur when the pivoting movements of the laparoscopic tool are performed around a point that does not coincide with the fulcrum, whereas contact forces on the distal end are called manipulation forces and occur when there is an interaction between the laparoscopic tool and the internal tissue of the patient. Both components of the contact force can occur simultaneously, but the force and moment measurement device compiles the measurement of the sum total of contact forces, therefore the system incorporates an algorithm for modelling the contact forces capable of separating the contributions to the measurement of reaction forces and manipulation forces. Thus, with a low magnitude in the contact force measurement, the relevant interaction is considered to occur in the fulcrum, in which case no command is transmitted to actuate the haptic device and the contact force measurement is used entirely in estimating the position of the fulcrum. In contrast, with a high magnitude in the contact force measurement, the relevant interaction is interpreted as being determined by the actuation of the distal end of the laparoscopic tool on the patient. In this case, the contact force measurement is used to actuate the haptic device for simulating the sensation of pressure of the laparoscopic tool on the user, maintaining the estimation of the fulcrum until the surgical tool stops exerting these high-magnitude contact forces.

The contribution of the reaction forces is used for precisely estimating the location of the fulcrum during movement of the laparoscopic tool. Since the manipulator is a servo actuated device without force reduction mechanisms and performs this movement around the fulcrum, an incorrect location of this position can give rise to injuries around the incision through which the laparoscopic tool is introduced into the patient. As a result, the estimation of the fulcrum is performed through a reaction force and moment equilibrium. This estimation is processed by a control element which is in charge of correcting the positioning of the laparoscopic tool, such that said tool is always aligned with the fulcrum in order for the force exerted on the patient at said point to thus be the minimum force.

During a surgical intervention, manipulation of the internal tissue of the patient performed by the laparoscopic tool can also be measured through the contribution of manipulation forces and moments. In order for the user to be able to have sensations of pressure similar to those the user would have if he or she directly manipulated the laparoscopic tool (without intermediation of the surgical robot), an algorithm for estimating the stiffness of the internal tissues of the patient processes the contribution of the manipulation forces (without taking into account the contribution of the reaction forces on the fulcrum point) to model a contact force such that it allows the user to perceive, through the haptic device, different degrees of stiffness of the internal tissue manipulated by the laparoscopic tools. These measurements are used in the actuators of the haptic device such that they move it in the direction opposite the movement, creating a sensation of pressure on the hand of the user.

Each element making up the entire remote teleoperation system of surgical robots through the console handled by the surgeon, whether physical control devices or algorithms, includes an additional layer of operating supervision, the purpose of which is to analyse that all the elements work correctly. Errors may occur at a local level in a device or control algorithm, or they may be the result of a poor interaction between several of the control devices or algorithms. The layer of supervision considers all the possible errors that may alter the normal operation of the remote teleoperation system of surgical robots and assigns them a risk index, such that with a low risk index the supervisor will properly modify the operation of the control device/devices and/or algorithms involved in the error to enable continuing with the intervention, whereas with a high risk the system will stop and the surgical robots will be manually removed by human assistants.

Other advantages and features of the invention will become apparent in view of the description provided below.

## Brief description of the drawings

To complement the description and for the purpose of helping to better understand the features of the invention according to a practical embodiment thereof, a set of figures is attached as an integral part of said description in which the following is depicted in an illustrative and non-limiting manner:

Figure 1 depicts a diagram of a surgical robotic system suitable for implementing the method. A surgical intervention by means of a teleoperated system is depicted, in which the surgical robot or slave element performs the surgical manoeuvres on the patient, whereas the surgeon sends the movement commands remotely to the surgical robot through the console or master element.

Figure 2 depicts a flowchart describing the relations between the different elements of the system controlling the surgical robot from the console.

Figure 3 schematically depicts a robotic arm and a laparoscopic surgical tool coupled to its distal end and depicts the methodology proposed for performing spherical navigation of laparoscopic tools.

Figure 4 shows the interaction between the laparoscopic tool coupled to the surgical robot and the two main points of contact with the patient: the skin and internal tissue.

Figure 5 shows a block diagram representing the control algorithm used for estimating the position of the fulcrum.

Figure 6 shows a block diagram representing the control algorithm in charge of modelling the force perceived by the surgical robot in order to reproduce it in the haptic device handled by the surgeon.

## Mode(s) of carrying out the invention

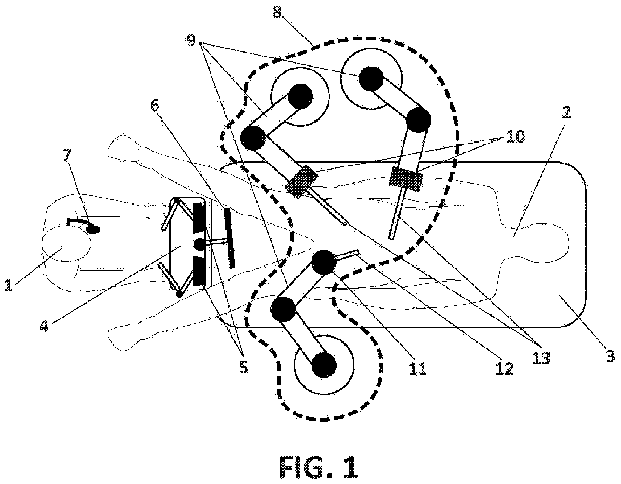

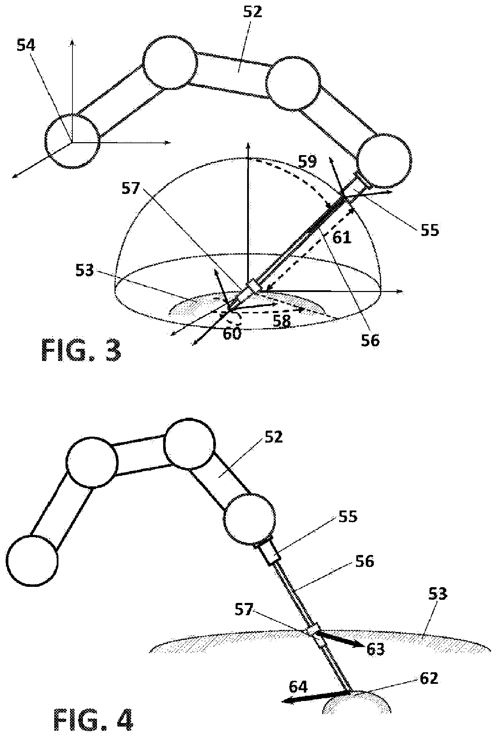

Figure 1 shows a diagram of a surgical robotic system implementing the method of the invention. Figure 1 depicts a minimally invasive surgical intervention. Examples of interventions in which minimally invasive surgery can be used are abdominal (cholecystectomy, nephrectomy, prostatectomy...), intracranial (tumour resection...) or trauma (arthroscopy, orthopaedic...) interventions, among others. Figure 1 depicts an abdominal laparoscopic intervention in which the surgeon 1 works on a console 4 from which he or she controls the movements of the surgical robot 8. The surgical robot 8 is that which operates on the patient 2 laying on the operating table 3. The console or interface 4, handled by a human user, acts as the master system. The surgical robot 8 manipulating the surgical tools 12, 13 (in this case, minimally invasive instruments) acts as the slave system. The surgical robot 8 is formed by three robotic units 9. Each of them has a robotic arm or manipulator arm. The surgical robot 8 can be formed by more or fewer robotic units 9. Each robotic unit has at least 6 degrees of freedom. The manipulator arm is explained in detail in relation to Figures 3 and 4. One of the robotic units is configured for having an endoscope 12 with a laparoscopic camera 11 and the other robotic unit or units are configured for having a minimally invasive instrument (such as a laparoscopic surgical tool) 13, strictly speaking. The terms "surgical tool", "laparoscopic tool" or "minimally invasive instrument" are used throughout this text in a general manner, such that they refer not only to surgical tools, strictly speaking, such as scalpels or clamps, but also to any supporting surgical or diagnostic equipment, such as endoscopes, cameras, etc. Each manipulator arm of each robotic unit has a sensor (force and moment measurement device) 10 coupled on its end effector which enables a measurement of the forces and moments of contact between the laparoscopic tool and the patient 2 to be obtained. The robotic unit 9 which has the endoscope 12 (and camera 11) also has a sensor 10 like the ones mentioned, although it is not illustrated in Figure 1. During use of the robotised surgical system, each robotic unit 9 is arranged close to the operating table 3 and surgical personnel guide the distal end of the manipulator arm (of each robotic unit 9) until introducing the surgical tool 12, 13 through a trocar which has previously been inserted through the skin of the patient 2 through an incision. That is, the minimally invasive instruments or surgical tools 12, 13 are introduced through the incision, by means of the trocar, into the patient. The manipulator arm is then ready to be used in the surgical operation.

The master system includes, in addition to the control console 4, at least one screen 6 which allows displaying the surgical area in two or three dimensions in time real as a result of the images taken by the camera 11 of the endoscope 12. The master system also includes an actuating device, not illustrated in Figure 1, by means of which the handling of surgical tools 12, 13 can be activated or deactivated. In a possible embodiment, the actuating device is implemented by means of one or more pedals. The master system also includes two servo actuated and sensorised mechanical devices (haptics) 5 which, on one hand, record the movements performed by the hands of the user for moving and orienting reference points determined by each haptic 5, and on the other hand enable a force to be transmitted to the hands of the user through the servo actuators in the hands of the user, in order for the user to be able to perceive the contact of the laparoscopic tools 13 with the patient 2.

Each robotic unit 9 of the slave system (surgical robot 8) comprises a robotic arm (i.e., a servo actuated and sensorised device) that is independent (of other robotic arms of other robotic units 9), at the distal end of which a surgical tool 12, 13 is coupled. The function of this tool is to reproduce the movements recorded by an associated haptic device 5 for said tool 12, 13 to move synchronously with the hand of the user. The haptic device 5 and the manipulator arm associated therewith can be separated from one another a certain distance and communicate through the transmission of electrical signals through a communications cable or wirelessly. Each manipulator arm of each surgical unit 9 has a force and moment measurement device (sensor) 10 coupled on its end effector (distal end of the manipulator arm) which enables a measurement of the forces and moments of contact between the surgical tool and the patient to be obtained, as described in detail below.

That is, the surgeon 1 can see the surgical area through the screen 6, which receives the image from the laparoscopic camera 11. The surgeon can move the haptic devices 5 with his or her hands, which movement is recorded and sent to the robotic units 9. The surgeon can also send voice commands recorded through a microphone 7 to move the robotic unit 9 holding the optic or endoscope 12. The movement of the robotic units 9 holding the laparoscopic tools 13 and/or the endoscope 12 (surgical tools in general) can cause force reactions in the patient which are measured with the respective force sensors 10. The movement mode of the robotic units 9 can be selected by the surgeon 1 according to two possible types: Mode "A" relates a movement of the haptic devices 5 with a movement of the distal end of the laparoscopic surgical tools 12, 13, whereas mode "B" relates a movement of the haptic devices 5 with a movement of the proximal end of the laparoscopic tools 12, 13.

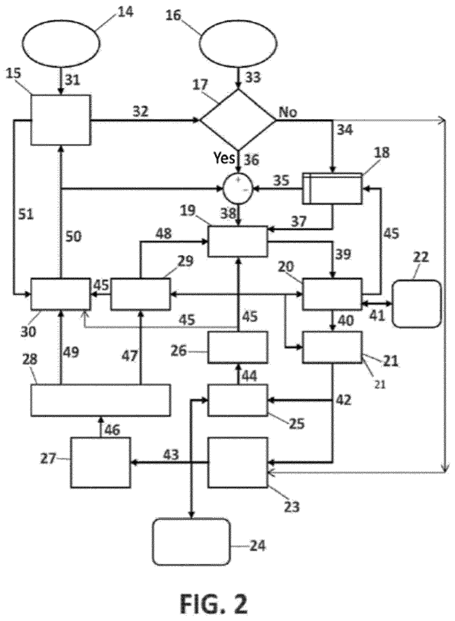

Figure 2 shows the flowchart of a control algorithm (or set of control algorithms) of the system described in Figure 1 for communications between one of the haptic devices 5 and one of the robotic units 9, which in Figure 2 is referenced as 23, according to a possible embodiment of the invention. This set of control algorithms is executed in the robotic unit, except the part relative to the haptic device 15 and the actuator 17, which is executed from the control unit or console 4 illustrated in Figure 1. Specifically, the algorithms are executed in computing or computer means comprising processing means, such as a microprocessor, processing unit, or any alternative conventional processing means, and conventional memory storage media. The hand of the surgeon 14 directs the movement of the haptic device 15, which is moved and oriented 31 around the axis of the laparoscopic tool (minimally invasive instrument) by the hand of the surgeon 14. The haptic device 15 transmits 32 to the actuating device 17 the absolute position $^{\{H\}}P_H$ and absolute torsion $\gamma_H$ of the haptic device 15, preferably by means of a signal filtered with a first-order low-pass Butterworth filter. Torsion is one of the three components of the orientation of the haptic device 15. The actuating device 17 is preferably implemented by means of a pedal or clutch pedal configured for being actuated by the foot of the surgeon 16. The foot of the surgeon 16 pushes or releases the clutch pedal 17, sending the signal indicating the presence or absence of actuation 33 on the actuating device 17.

If the pedal 17 is not pushed 34, on one hand the references 18 of the absolute position $^{\{H\}}P_H{}^P$ of the haptic device 15, the absolute torsion $Y_H{}^P$ of the haptic device 15 (received by means of the signal 32) and the homogeneous matrix $^{\{B\}}T_{\{R\}}{}^P$ of the end effector 55 (illustrated in Figures 3 and 4) of the manipulator arm of the robotic unit are updated (and saved in the memory) to make the difference 38 between the absolute position $^{\{H\}}P_H$ and absolute torsion $\gamma_H$ 32 and a reference position and torsion $^{\{H\}}P_H{}^P$, $\gamma_H{}^P$ 35 of the relative movement of the haptic device 15 equal to 0, and on the other hand, a not pushed pedal signal is sent to the robotic unit 23 to establish the free/manual movement mode, such that an assistant can manipulate it with the hands. It must be observed that the homogeneous matrix is a mathematical term describing a position and orientation of a Euclidean reference system referring to another base reference system in a 4x4 matrix, such that the 3x3 upper left submatrix describes the orientation relative to the base system, with each column vector being one of the axes of the system, whereas the 3x1 vector on the right side of the matrix corresponds to the Cartesian position relative to the base system.

If the pedal is indeed pushed 36 then the differences $\Delta P_H$, $\Delta\gamma_H$ between the absolute position and absolute torsion $^{\{H\}}P_H$, $Y_H$ 32 of the haptic device 15 and the reference position and torsion $^{\{H\}}P_H{}^P$, $\gamma_H{}^P$ saved in the memory 18 are applied for obtaining the relative movement 38 of the haptic device 15. On one hand, scaling factors of position $K_P$ and torsion $K_y$ established by the surgeon to increase movement precision, and on the other hand, a factor of reduction $K_s$ the expression of which is a sigmoid function which depends on the force feedback $F_H$ 50 obtained from a stiffness estimator 30 described in detail below, are applied to the difference $\Delta P_H$. These scaling factors of position and torsion and of reduction can be applied both in a navigation model 19 like in the preceding step, in which the differences between the absolute position and torsion of the haptic device 15 and the reference position and torsion saved in the memory are applied for obtaining the relative movement 38 of the haptic device 15. This force feedback $F_H$ 50 is parameterised by a value $\sigma$ indicating the minimum value of $K_s$, a value w indicating for which force feedback $F_H$ 50 the maximum value of $K_s$ is produced, and a value c indicating the steepness of the upslope/downslope of $K_s$, the purpose of which consists of reducing the speed in the movement of the surgical tool when it comes into contact with the internal tissue of the patient to thus improve stability of the control algorithm for controlling the feedback of forces resulting from contact with the tissue of the patient to the surgeon:

$$K_S = \sigma + \frac{1 - \sigma}{1 + e^{c(|F_H| - \omega)}}$$

$$\Delta\gamma_H = K_\gamma(\gamma_H - \gamma_H^P) \qquad\qquad [1]$$

$$\Delta\boldsymbol{P}_H = K_S K_P({}^{\{H\}}\boldsymbol{P}_H - {}^{\{H\}}\boldsymbol{P}_H^P)$$

Figure 2 also includes a navigation model 19 comprising an algorithm for programming the next spherical position of the distal end of the surgical tool. For programming the spherical position, the navigation model 19 receives the relative movement 38 of the haptic device 15, which is already scaled, the homogeneous matrix ${}^{\{B\}}T_{\{R\}}{}^P$ 37 of the end effector 55 of the robotic arm with the reference Cartesian position and orientation (torsion) at the time of pushing the pedal 17, the homogeneous matrix with the modelled Cartesian position and orientation 45 of the robot (model of the robot 25) for calculating the next position of the distal end of the laparoscopic tool or minimally invasive instrument, and the estimation of the position of the fulcrum 48 provided by a fulcrum estimator 29 for obtaining the next programmed position 39 of the end effector 55 of the robotic arm. Throughout the description of Figure 2, where the "model of the robot 25" is mentioned, to be precise, this expression refers to the "model of the robotic unit 25", but on occasions the term "robot" is used for the sake of simplicity.

To explain in further detail the navigation model 19, Figure 3 depicts the robotic arm or manipulator arm 52 of a robotic unit 9 (23 in Figure 2). In Figure 3, the manipulator arm 52 is performing spherical movements around the fulcrum point 57 located on the skin of the patient 53. The fulcrum point 57 is the point of insertion of the surgical tool into the patient. The fulcrum point is located in the incision made in the skin of the patient. Figure 3 illustrates the problem with spherical navigation solved by the navigation model 19. A coordinate system {/} at the fulcrum point 57 is defined. The axes of the coordinate system {/} remain parallel to those of a reference system {B} associated with the base 54 of the robotic unit 9. A reference system {R} associated with the end effector 55 of the manipulator arm 52 and a reference system {T} associated with the distal end of the laparoscopic tool 56, both with their main directional axes parallel to one another, are also defined. The location of the end effector 55 of the manipulator arm 52 relative to the fulcrum point 57 is established through the spherical coordinates defined as orientation angle $\alpha$ 58, altitude angle $\beta$ 59, orientation of torsion $y$ 60 around its own axis and external distance p 61 or distance along the instruments spanning from the centre of rotation of the end effector 55 to the fulcrum point 57. It must be observed that angles $\alpha$ and $\beta$ can be obtained by direct reading of the internal sensors of the robot, which does not occur with the external distance p. It must be observed that the internal sensors of the robot are not the sensors 10 located in the end effector of the manipulator arm (see Figure 1), but rather they are sensors internally assembled in the actuators of the motor of each robotic unit for taking measurements of their position, speed, etc. These internal sensors do not fall within the scope of the present invention.

Navigation in a spherical coordinate system is established by means of the vector of spherical components ($\alpha$, $\beta$, p, $\gamma$), which can be obtained based on the relationship between the homogeneous matrix ${}^{\{I\}}T_{\{R\}}$ expressed in Cartesian and spherical coordinates, which defines the Cartesian position and orientation of the end effector 55 of the manipulator arm 52 relative to the fulcrum point 57. This homogeneous matrix ${}^{\{I\}}T_{\{R\}}$ can be calculated based on its relationship with the homogeneous matrix ${}^{\{B\}}T_{\{R\}}$ of the end effector 55 of the manipulator arm 52 relative to the base 54 and with the homogeneous matrix ${}^{\{B\}}T_{\{I\}}$ of the fulcrum point 57 relative to the base 54. The homogeneous matrix ${}^{\{B\}}T_{\{R\}}$ of the end effector 55 of the manipulator arm 52 relative to the base 54 is obtained 45 by means of the forward kinematics algorithm 26 (which will be explained below, see Figure 2); the homogeneous matrix ${}^{\{B\}}T_{\{I\}}$ of the fulcrum point 57 relative to the base 54 is obtained 48 by means of the fulcrum estimator algorithm 29 (see Figure 2):

$${}^{\{I\}}\mathbf{T}_{\{R\}} = ({}^{\{B\}}\mathbf{T}_{\{I\}})^{-1} \cdot {}^{\{B\}}\mathbf{T}_{\{R\}}$$

$${}^{\{I\}}\mathbf{T}_{\{R\}} = \begin{bmatrix} \cos\alpha\cos\beta & -\sin\alpha & \cos\alpha\sin\beta & \rho\cos\alpha\sin\beta \\ \sin\alpha\cos\beta & \cos\alpha & \sin\alpha\sin\beta & \rho\sin\alpha\sin\beta \\ -\sin\beta & 0 & \cos\beta & \rho\cos\beta \\ 0 & 0 & 0 & 1 \end{bmatrix} \cdot \begin{bmatrix} \cos\gamma & -\sin\gamma & 0 \\ \sin\gamma & \cos\gamma & 0 \\ 0 & 0 & 1 \\ 0 & 0 & 0 \end{bmatrix} \quad [2]$$

Using this principle, the homogeneous matrix of the next position ${}^{\{B\}}\mathbf{P'}_R$ and orientation ${}^{\{B\}}\mathbf{z'}_{\{R\}}$ of the axis of the laparoscopic tool 39 can be calculated based on the position ${}^{\{B\}}\mathbf{P}_R{}^P$ and orientation ${}^{\{B\}}\mathbf{z}_{R}{}^P$ of the reference axis 37 of the end effector 55 of the robotic arm, the length of the laparoscopic tool L and the increase in movement of the haptic device $\Delta\mathbf{P}_H$ 38 according to the movement mode established for the laparoscopic tool. Thus, in mode A, in which the movements of the haptic device 15 correspond to movements of the distal end of the laparoscopic tool, the geometric relationships are of the Cartesian type, whereas in mode B, in which the movements of the haptic device 15 correspond with spherical movements *(α', β', ρ')* of the proximal end of the laparoscopic tool relative to the reference spherical positions *(α^P, β^P, ρ^P)*, the geometric relationships are of the spherical type:

Mode A

$$^{\{B\}}\mathbf{P'}_{\mathbf{T}} = {}^{\{B\}}\mathbf{P}^{\mathbf{P}}_{\mathbf{R}} + L\,{}^{\{B\}}\mathbf{z}^{\mathbf{P}}_{\{\mathbf{R}\}} + \Delta\mathbf{P}_{\mathbf{H}}$$
$$^{\{B\}}\mathbf{z'}_{\{\mathbf{R}\}} = {}^{\{B\}}\overline{P_I P'_T}$$
$$^{\{B\}}\mathbf{P'}_{\mathbf{R}} = {}^{\{B\}}\mathbf{P'}_{\mathbf{T}} - L\,{}^{\{B\}}\mathbf{z'}_{\{\mathbf{R}\}}$$

[3]

Mode B

$$\alpha' = \alpha^P + \Delta x_H$$
$$\beta' = \beta^P + \Delta y_H$$
$$\rho' = \rho^P + \Delta z_H$$

That is, in mode B, the spherical coordinates vector of the next programmed position and orientation 40 is calculated (see "kinematic supervisor 20" of Figure 2).

In Figure 2, the kinematic supervisor 20 represents an algorithm which verifies, based on the current position and orientation 45 of the end effector of the model of the robot 25, the position and orientation 39 programmed by the navigation model 19 and the position and orientation 41 of other robotic units 22, if any, that the programmed position and orientation 39 for the end effector 55 of the robotic unit comply with certain safety criteria, keeping the laparoscopic tool within a valid work area. Some possible safety criteria consist of the distal end of the surgical tool remaining within the visible surgical area, or the proximal end of the surgical tool (end effector 55 of the robotic unit 23) remaining at a minimum distance from other robotic units, or the surgical tool being able to perform spherical navigation within a subspace such that it must comply with physical limits, such as not introducing the end effector 55 of the robotic unit 23 past the fulcrum point 48 estimated by the fulcrum estimator 29 such that the external distance is nil, or the spherical altitude angle does not exceed 90° since the distal end of the surgical tool would try to come out of the skin of the patient. In the event of failing to comply with any of these kinematic criteria, the kinematic supervisor 20 limits the programmed position and orientation 39 of the end effector 55 of the robotic unit 23 (programmed by the navigation model 19) to not advance outside of the work area.

In Figure 2, the inverse kinematics 21 represents an algorithm which receives the current position and orientation 45 of the end effector of the model of the robot (obtained by the forward kinematics algorithm 26, which is explained below) and the spherical coordinates vector of the next programmed position and orientation 40 restricted by the kinematic supervisor 20 of the end effector of the robotic unit 23, information is converted into the articular speeds and positions 42 required in order for each degree of freedom of the robotic unit 23 moved by an actuator to locate the next programmed position and orientation 40 of the end effector of the robotic unit 23.

The actuators of the robotic unit 23 receive the articular speeds and positions 42 coming from the inverse kinematics algorithm 21. The articular speeds and positions 42 are processed by a position and speed control based on gains to ensure that the programmed Cartesian path calculated by the navigation model 19 are followed. The programmed Cartesian path will reproduce the interaction between the laparoscopic tools and the patient 24 (interaction desired by the surgeon). As output of the robotic unit 23, the positioning and speed sensors of the robotic unit itself measure 43 the articular speed and position parameters.

The model of the robot 25 represents an algorithm which calculates the dynamics of the actuators of the robotic unit 23 by means of a model of the actual behaviour of the actuators, such that with respect to the same setpoints of articular speed and position 42 desired for each actuator, obtained from inverse kinematics 21, the articular speeds and positions 43 of the robotic unit 23 evolve similarly to the articular speeds and positions 44 in the model of the robot 25. The function of the model of the robot 25 is to reduce the instabilities caused by the differences between the sampling frequency at which the state signals of the articular speed and position 43 of the robotic unit 23 in the slave system are updated and the sampling frequency at which the state signal of the position of the haptic device 15 is updated and the state 50 of the actuators of the haptic device 15, provided by the stiffness estimator 30, is updated as well. To minimise the error between actual articular speeds and positions 43 and modelled articular speeds and positions 44, the model of the robot 25 receives the articular speed and position 43 of the robotic unit 23 to update its internal state variables at each sampling time established by the signals sent by the robotic unit 23.

Forward kinematics 26 represents an algorithm which converts the articular position 44 of the model of the robot 25 into Cartesian coordinates 45, which are used by the navigation model 19 in order to know the reference Cartesian position of the robotic unit 23 based on which it calculates the programmed position and orientation 39 (for example the spherical navigation path).

The force sensor 27 moves integrally with the end effector of the robotic unit 23, so its position and orientation are determined by the articular state 43 thereof. The force sensor 27 includes algorithms to offset both the gravitational moment and the moment of inertia exerted by the actuators and sensors integrated in the device for coupling the laparoscopic tool and the laparoscopic tool itself. The offset force and moment measurement in the force sensor 27 is converted into the form of an electrical signal that is filtered, preferably by means of a first-order low-pass Butterworth filter (a filtered signal $^{\{R\}}F$ being obtained), to enable being sent 46 to the console 4 through the communication channel between the robotic unit 23 and the console 4.

The interaction model 28 represents an algorithm which determines what percentage of the measurement of the offset forces $^{\{R\}}\mathbf{F}$ 46 is contributed as a result of the interaction with the fulcrum $^{\{R\}}\mathbf{FI}$ 47 or with the internal tissue of the patient $^{\{R\}}\mathbf{F_T}$ 49 by means of contribution parameters $\lambda_I$, $\lambda_T$, respectively. This distinction is made based on the magnitude of the offset force measurement 46 with a parameterised sigmoid function for each contribution according to the following qualitative criteria:

If the measurement of the offset forces $^{\{R\}}\mathbf{F}$ 46 is low, then virtually all the force results from the interaction with the fulcrum, such that $47 \approx 46$ and $49 \approx 0$.

If the measurement of the offset forces $^{\{R\}}\mathbf{F}$ 46 is high, then virtually all the force results from the interaction with the internal tissue of the patient, such that $47 \approx 0$ and $49 \approx 46$.

In a preferred embodiment, the contribution parameters are calculated based on the following expression:

$$\lambda_I = \frac{1}{1+e^{c(|F-1|-\omega)}} \qquad\qquad {}^{\{R\}}\mathbf{F_I} = \lambda_I\,{}^{\{R\}}\mathbf{F}$$
$$\longrightarrow \qquad\qquad\qquad\qquad [4]$$
$$\lambda_T = \frac{1}{1+e^{c(1-F-\omega)}} - \lambda_I \qquad {}^{\{R\}}\mathbf{F_T} = \lambda_T\,{}^{\{R\}}\mathbf{F}$$

Parameter $\lambda_I$ is subtracted from $\lambda_T$ to cancel the effect of the measurement of the offset forces $^{\{R\}}\mathbf{F}$ 46 in the force of interaction with the internal tissue of the patient 49 when the measurement of offset forces $^{\{R\}}\mathbf{F}$ 27 reports low values. Parameter $\lambda_I$ adopts values close to nil when the measurement of offset forces $^{\{R\}}\mathbf{F}$ 46 is high. Parameter w indicates where the maximum sigmoid occurs, whereas c serves to increase or decrease upslope/downslope of the sigmoid.

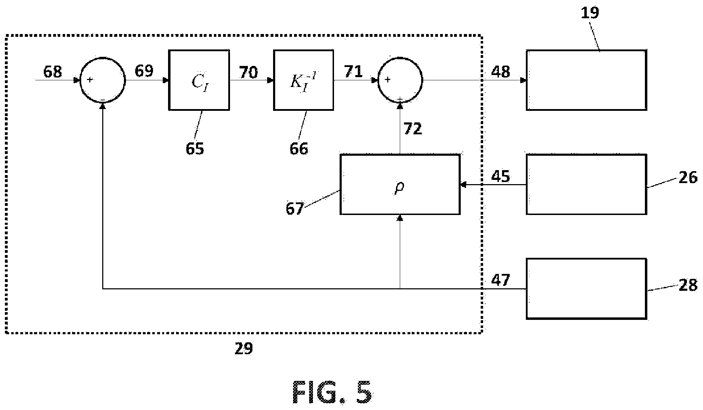

The fulcrum estimator 29 represents an algorithm which, on one hand, is in charge of estimating the external distance p along the axis of the laparoscopic tool at which the fulcrum point of the Cartesian position 45 (modelled by forward kinematics 26) of the end effector of the robotic unit 23 is located. This external distance is calculated by applying signal force and moment equilibrium equations with the offset force and moment measurement resulting from the interaction with the fulcrum 47. A Cartesian movement of the fulcrum in the direction perpendicular to the axis of the laparoscopic tool obtained based on a force control algorithm based on impedances, which uses as a feedback reference the offset force and moment measurement resulting from the interaction with the fulcrum 47, is added to this estimation of the external distance, obtaining as a result the estimated Cartesian position of the fulcrum 48. Figure 5 details the control algorithm used for estimating the position of the fulcrum 48.

The method proposed for obtaining the estimation of the fulcrum point 48 depends on the type of orientation mechanism of the laparoscopic tool installed in the end effector of the robotic unit. In a preferred embodiment, this orientation mechanism of the laparoscopic tool (or minimally invasive instrument) is performed by means of direct actuation and is discussed in further detail according to the illustration of Figure 4. If the actual location of the fulcrum point $^{\{R\}}\mathbf{P_I}$ 57 (see Figures 3 and 4) is different from the estimated location, the error in the positioning $^{\{R\}}\Delta\mathbf{I}$ of the fulcrum generates an unwanted abdominal force $^{\{R\}}\mathbf{F_I}$ 63 on the abdominal wall 53. The abdominal force $^{\{R\}}\mathbf{F_I}$ 63 can be measured with the force sensor coupled in the end effector 55 of the robot. If the scalar magnitude of the force $F_I$ and moment $M_I$ of interaction with the fulcrum 63 is virtually nil, then the estimated fulcrum coincides with the actual fulcrum, and the estimation of the fulcrum point 57 does not have to be updated. Otherwise, the external distance p can be calculated by means of the following expression obtained based on the equilibrium of moments:

$$\rho = \frac{\dot{M}_I}{F_I} \qquad\qquad [5]$$

On the other hand, for the interaction between the laparoscopic tool 56 and the abdominal wall 53, a linear elastic behaviour is assumed by means of the gain $K_I$, which relates the vector of the force $^{\{R\}}\mathbf{F_I}$ of interaction with the fulcrum 63 with the elongation $^{\{R\}}\Delta\mathbf{I}$ produced on this surface:

$${}^{\{R\}}\mathbf{F_I} = K_I\,{}^{\{R\}}\Delta\mathbf{I} \qquad\qquad [6]$$

The gain $K_I$ is obtained in a general manner based on experimental testing in which the force of interaction with the fulcrum $F_I$ 63 for known movements $\Delta l$ is measured. A gain $C_I < 1$, the value of which is chosen to meet the rapid response criteria for a feedback loop and to ensure stability of the control algorithm is applied to this result. The estimation of the position of the fulcrum point ${}^{\{R\}}\mathbf{P_I}$ 57 is thereby determined by the sum of the elongation of the abdominal wall recorded by the measurement of the force of interaction with the fulcrum 63 and the estimation of the external distance to the fulcrum obtained by equilibrium of moments and located in the direction of the axis ${}^{\{R\}}\mathbf{z}$ of the end effector 55 of the manipulator arm 52:

$$ {}^{\{R\}}\mathbf{P_I} = \frac{C_I}{K_I}{}^{\{R\}}\mathbf{F_I} + \rho\ {}^{\{R\}}\mathbf{z} \qquad [7] $$

Figure 5 shows the control algorithm used for estimating the position of the fulcrum 48 according to the method set forth above. The external distance p 67 is calculated based on the expression [5], i.e., the external distance p 67 is calculated based on the current Cartesian orientation ${}^{\{R\}}\mathbf{z}$ 45 coming from the forward kinematics 26 and the contribution of the forces ${}^{\{R\}}\mathbf{F_I}$ and moments ${}^{\{R\}}\mathbf{M_I}$ of interaction with the abdominal wall 47 coming from the interaction model 28. This contribution of the forces ${}^{\{R\}}\mathbf{F_I}$ and moments ${}^{\{R\}}\mathbf{M_I}$ of interaction with the abdominal wall 47 is provided as feedback, being subtracted from a desired reference force ${}^{\{R\}}\mathbf{F_0}$ 68 for the interaction with the abdominal wall. The result 69 of this subtraction is weighted with the control gain $C_I$ 65, and the result of this weighting 70 is converted from a magnitude in forces to a magnitude in distances with conversion factor $K_I$ 66. The result of that conversion 71 is added to the estimation vector of the external distance $\mathbf{p}^{\{R\}}{}_{\mathbf{z}}$ 72 for finally obtaining the Cartesian position ${}^{\{R\}}\mathbf{P}$, of the fulcrum point 48 and sending it to the navigation model 19 for programming the spherical navigation.

In Figure 2, the stiffness estimator 30 represents an algorithm which is in charge of estimating in a dynamic manner the stiffness of the tissue in contact with the distal end of the tool by means of the offset force and moment measurement resulting from the interaction with the internal tissue of the patient 49 (obtained by the interaction model 28), such that the relationship between the force and the movement of interaction with the internal tissue of the patient can be modelled with a linear system. This estimation of the stiffness of the internal tissue of the patient is part of a least squares estimation algorithm, the purpose of which is to stabilise the value of the stiffness of the internal tissue with the smallest possible delay, whereby the perception of contact of the hands of the surgeon 14 through the haptic devices 15 can be distinguished between solid and soft objects. With this estimation of the stiffness, a simulated reaction force 50, proportional to the movement 51 of the haptic device 15 performed by the hand of the surgeon 14, is calculated taking as a reference the position of the haptic device 15 in which a force and moment measurement 49 resulting from the interaction with the internal tissue of the patient 49 was first detected. That simulated reaction force 50 is scaled so as to perceive the contact in the hands of the surgeon as a natural reaction, and is subsequently sent to the actuators of the haptic device 15.

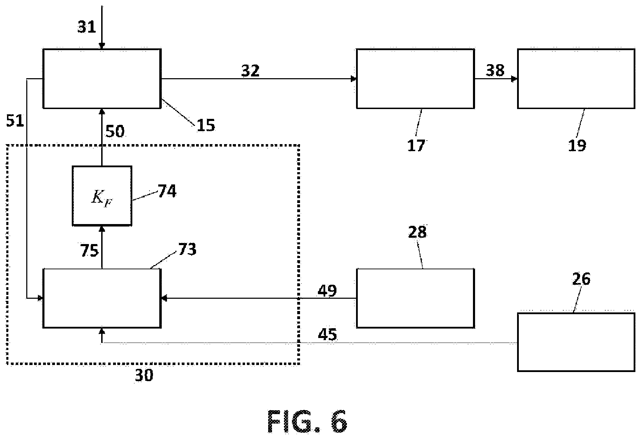

The control algorithm of the stiffness estimator 30 of Figure 2 is detailed in Figure 6 and is in charge of modelling the force perceived by the robotic unit as a simulated force ${}^{\{H\}}\mathbf{F_H}$ 50 acting in the direction opposite the movement of the haptic device 15. This force is simulated by means of an elastic-linear force reaction model 73 with dynamic stiffness $K_T$, the point of equilibrium of which is based on the last haptic position ${}^{\{H\}}\mathbf{P_H}^0$ and the current haptic position of which is ${}^{\{H\}}\mathbf{P_H}$ 32. Once the force 75 has been modelled by the force reaction model 73, a scaling factor $K_F < 1$ 74 is applied to improve system stability. The following expression represents the simulated force obtained 50:

$$ {}^{\{H\}}\boldsymbol{F}_H = -K_F K_T \left( {}^{\{H\}}\boldsymbol{P}_H - {}^{\{H\}}\boldsymbol{P}_H^0 \right) \qquad [8] $$

The dynamic stiffness $K_T$ is a variable parameter which enables different resistances to be perceived in the haptic 15 depending on the material in contact with the laparoscopic tool. For this reason, it is necessary to determine this magnitude as a function of the measurements of forces ${}^{\{R\}}\mathbf{F_T}$ of interaction with the internal tissue 49 obtained from the actual surroundings of the robotic unit 23 through the interaction model 28. Also for that reason, it is necessary to determine dynamic stiffness $K_T$ as a function of the current position of the distal end of the tool ${}^{\{R\}}\mathbf{P_T}$ 45 (it must be observed that based on the homogeneous matrix describing the position and orientation of the robot, both the vector of the current Cartesian orientation ${}^{\{R\}}\mathbf{z}$ and the current position of the distal end of the tool ${}^{\{R\}}\mathbf{P_T}$ can be extracted) obtained from the forward kinematics 26 of the surgical robot and the distal position of the end of the tool in the moment of contact ${}^{\{R\}}\mathbf{P_T}^0$:

$$ K_T = \frac{F_T}{\left\| {}^{\{R\}}P_T - {}^{\{R\}}P_T^0 \right)\right\|} = \frac{F_T}{\Delta P_T} \qquad [9] $$

Since this result is instantaneous and highly variable, particularly in the vicinity of the point of contact by amplification of the noise from the signal which records small deformations $\Delta P_T$, a recursive least squares estimation algorithm is applied. With this method, a stable value of $K_T$ can be obtained after a few sampling cycles. The recursive least squares algorithm consists of the following steps:

Start the method (iteration N= 0):

$$Ke(0) = 0 \qquad [10]$$

$$C(0) = \varphi \qquad [11]$$

Where $\varphi$ must be a sufficiently large number (the higher it is, the faster $K_T$ varies relative to each iteration), and C is a parameter of the algorithm which evolves with the iterations.

In the N-th iteration, calculate the new estimation of $K_T$ according to:

$$K_T(N + 1) = K_T(N) + (F_T - \Delta P_T(N) \cdot K_T(N)) \frac{\Delta P_T(N) \cdot C(N)}{1 + \Delta P_T^2(N) \cdot C(N)} \qquad [12]$$

Update C:

$$C(N + 1) = \frac{C(N)}{1 + \Delta P_T^2(N) \cdot C(N)} \qquad [13]$$

Return to step 2.

In summary, the method of the invention overcomes the main limitations detected in the state of the art as has been explained:

Relative to conventional control methods, which allow the surgeon to perceive the interaction of forces between the surgical tools and the patient, but such proposals do not take into account the overlapping of forces exerted by the laparoscopic tool on the point of insertion and manipulation forces relative to the internal tissue of the patient: The described method takes into account in that force and moment measurement between the surgical tools and the patient what percentage of the measurement results from the interaction with the fulcrum point and what percentage of the measurement results from the interaction with the internal tissue of the patient. Contact forces on the fulcrum (reaction forces) occur when the pivoting movements of the laparoscopic tool are performed around a point which does not coincide with the fulcrum, i.e., when an erroneous estimation of the point of insertion takes place. Contact forces on the distal end (manipulation forces) occur when an interaction between the laparoscopic tool and the internal tissue of the patient takes place. Both components of the contact force can be produced simultaneously, but the force and moment measurement device compiles the measurement of the sum total of the contact forces. The method models the contact forces and is capable of separating the contributions of the reaction forces and the manipulation forces to the measurement. Each manipulator arm has a force control system which uses, on one hand, the manipulation force for obtaining an estimation of the stiffness of the internal tissue of the patient for modelling a contact force with a hard or soft tissue which is subsequently provided as feedback to the haptic interface, and on the other hand, the reaction force for obtaining a better estimation of the position of the point of insertion for minimising the magnitude of said force by means of programming paths of the laparoscopic tool around the actual point of insertion.

As regards the proposals for the control of movements of laparoscopic tools handled by a surgical robot which depend on mechanisms which either produce certain play/imprecision in the positioning of surgical tools, or else require offline recalibration, both at the beginning of the intervention and in the case of movement of the fulcrum point resulting from factors such as a possible movement of the patient on the operating table: The described method estimates the position of the fulcrum point with equation [7]. With this estimation of the fulcrum, a control algorithm (Figure 5) which moves the laparoscopic tool of the robot in order for it to be aligned along the fulcrum is applied, such that it minimises the force exerted on the abdominal wall of the patient. Furthermore, this estimation of the position of the fulcrum is used for performing spherical navigation and in order for the new movements of the laparoscopic tool to be performed around this point.

Finally, relative to conventional surgical robots which only allow one type of correspondence between the movement of the haptic devices and of the laparoscopic tools, such that the position of the end effector of the haptic device can only correspond can to the position of the distal end of the laparoscopic tool handled by the surgical robot: The described method allows selecting, through an interface of the control console (for example through a touch screen), a type of movement of the minimally invasive instrument: a first type in which the movement of the haptic device is related to the movement of the distal end of the minimally invasive instrument; or a second type in which the movement of the haptic device is related to the movement of the effector of the manipulator arm (i.e. with the proximal end of the minimally invasive instrument), such that the pivoting movements of a manual minimally invasive instrument are simulated through the haptic device.

**Claims**

1. A robotic system for minimally invasive surgery, wherein the robotic system comprises:

   a control console (4) comprising at least one actuating device (17) and a haptic device (5, 15) which in turn comprises one or more positioning sensors and servo actuators, and at least one robotic unit (9, 23) comprising:

   a manipulator arm (52),
   an effector (55) arranged at the distal end of said manipulator arm (52), said effector (55) being equipped with at least one force and moment sensor (10, 27),
   at least one actuator,
   and a minimally invasive instrument (12, 13, 56) coupled to said effector (55), wherein the distal end of said minimally invasive instrument (12, 13, 56) is configured for being introduced into a cavity of the body of a patient through a fulcrum point (57),
   the robotic system comprising computing means configured to carry out the following steps:

   programming a position and orientation (39) of said effector (55) based on a relative displacement (38) of said haptic device (15), on reference coordinates (37) of said effector (55), on coordinates (45) of the effector (55) according to a model (25) of the robotic unit (23), and on an estimation (48) of the position of a fulcrum point;
   based on said coordinates (45) of the effector (55) according to said model (25) of the robotic unit (23) and on the programmed position and orientation (39) of the effector (55), obtaining articular speeds and positions (42) required so that each degree of freedom of the robotic unit (23) moved by said actuator, together, makes it possible to reach the next programmed position and orientation (39) of the effector (55);
   moving the effector (55) by means of said at least one actuator according to said articular speeds and positions (42);
   measuring (46) by means of said at least one force and moment sensor (10, 27) coupled to said effector (55), forces and moments exerted by said effector (55) and by said minimally invasive instrument (13, 56) coupled thereto when this movement is performed;
   determining (28) what percentage of said measurement (46) of forces and moments is contributed as a result of the interaction with the fulcrum point (47) or as a result of the interaction with the internal tissue of the patient (49), wherein this determination is made based on the magnitude of said measurement (46) by applying a parameterised sigmoid function for each contribution;
   estimating (48) the position of the fulcrum again, where said estimation (48) is performed based on the contribution resulting from said interaction with the fulcrum point (47) and on said coordinates (45) of the effector (55) according to said model (25) of the robotic unit (23);
   estimating (50) the stiffness of the tissue in contact with the distal end of the minimally invasive instrument (13, 56) and calculating a simulated reaction force (50);
   sending this simulated reaction force (50) to the at least one servo actuator of the haptic device (15) for it to be provided to the hand of the surgeon (14).

2. The system according to claim 1, wherein said coordinates (45) of the effector (55) according to said model (25) of the robotic unit (23) are obtained from an articular position (44) of said model (25).

3. The system according to any one of the preceding claims, wherein said relative displacement (38) of said haptic device (15) is obtained from the difference between an absolute position and absolute torsion (32) of the haptic device (15) and a reference position and torsion (18).

4. The system according to claim 3, wherein scaling factors of position $K_p$ and torsion $K_\gamma$ established by the surgeon to increase movement precision, and a reduction factor $K_s$ related to said simulated reaction force (50), are applied to said difference between an absolute position and absolute torsion (32) of the haptic device (15) and a reference position and torsion (18).

5. The system according to any one of the preceding claims, wherein to perform said estimation of the fulcrum point (48), the computing means is further configured for estimating the external distance p along the axis of said minimally invasive instrument (13, 56) at which the fulcrum point is located relative to the position (45) of the effector (55) according to said model (25) of the robotic unit (23).

6. The system according to any one of the preceding claims, wherein said estimation (50) of the stiffness of the tissue in contact with the distal end of the minimally invasive instrument (13, 56) and calculation of a simulated reaction force (50) are performed based on the contribution resulting from the interaction with the internal tissue of the patient (49), on said coordinates (45) of the effector (55) according to said model (25) of the robotic unit (23), and on the displacement (51) of the haptic device (15) performed by the hand of the surgeon (14).

7. The system according to any one of the preceding claims, wherein said calculation of a simulated reaction force (50) is performed based on the following expression:

$$\phantom{}^{\{H\}}\mathbf{F_H} = -K_F K_T \left( \phantom{}^{\{H\}}\mathbf{P_H} - \phantom{}^{\{H\}}\mathbf{P_H^0} \right)$$

where $\phantom{}^{\{H\}}\mathbf{F_H}$ represents the simulated force (50), $K_F$ is a scaling factor, with $K_F < 1$, $K_T$ is the dynamic stiffness, $\phantom{}^{\{H\}}\mathbf{P_H}$ is the current haptic position (32) and $\phantom{}^{\{H\}}\mathbf{P_H^0}$ is the last haptic position.

8. The system according to any one of the preceding claims, wherein the computing means is further configured for: based on the position and orientation (45) of the effector (55) according to said model (25) of the robotic unit (25), on the programmed position and orientation (39) and on the position and orientation (41) of other robotic units (22), if any, verifying that the programmed position and orientation for the final effector (55) of the robotic unit (9, 23) comply with safety criteria, and restricting (40) said position and orientation should they fail to comply with said criteria.

9. The system according to any one of the preceding claims, wherein the computing means is configured for: through an interface of the control console (4), selecting a type of movement of the minimally invasive instrument (13, 56): a first type in which the displacement of the haptic device (15) is related to the displacement of the distal end of the minimally invasive instrument (13, 56); or a second type in which the displacement of the haptic device (15) is related to the displacement of the effector (55) of the manipulator arm, such that the pivoting movements of a manual minimally invasive instrument (13, 56) are simulated through the haptic device (15).

10. The system according to any one of the preceding claims, wherein the computing means is configured to perform any of the preceding steps if said actuating device (16) is pushed or actuated.

11. The system according to any one of the preceding claims, wherein the actuating device (16) is a pedal.

12. A computer software product comprising instructions/computer program code to cause the device of claim 1 to execute the following steps:

a position and orientation (39) of said effector (55) based on a relative displacement (38) of said haptic device (15), on reference coordinates (37) of said effector (55), on coordinates (45) of the effector (55) according to a model (25) of the robotic unit (23), and on an estimation (48) of the position of a fulcrum point; based on said coordinates (45) of the effector (55) according to said model (25) of the robotic unit (23) and on the programmed position and orientation (39) of the effector (55), obtaining articular speeds and positions (42) required so that each degree of freedom of the robotic unit (23) moved by said actuator, together, makes it possible to reach the next programmed position and orientation (39) of the effector (55); moving the effector (55) by means of said at least one actuator according to said articular speeds and positions (42);

measuring (46) by means of said at least one force and moment sensor (10, 27) coupled to said effector (55), forces and moments exerted by said effector (55) and by said minimally invasive instrument (13, 56) coupled thereto when this movement is performed;

determining (28) what percentage of said measurement (46) of forces and moments is contributed as a result of the interaction with the fulcrum point (47) or as a result of the interaction with the internal tissue of the patient (49), wherein this determination is made based on the magnitude of said measurement (46) by applying a parameterised sigmoid function for each contribution;

estimating (48) the position of the fulcrum again, where said estimation (48) is performed based on the contribution resulting from said interaction with the fulcrum point (47) and on said coordinates (45) of the effector (55) according to said model (25) of the robotic unit (23);

estimating (50) the stiffness of the tissue in contact with the distal end of the minimally invasive instrument (13, 56) and calculating a simulated reaction force (50);

sending this simulated reaction force (50) to the at least one servo actuator of the haptic device (15) for it to be provided to the hand of the surgeon (14).

**13.** A computer-readable medium having stored thereon the computer program of claim 12.

**Patentansprüche**

**1.** Robotersystem für minimal invasive Chirurgie, wobei das Robotersystem umfasst:

eine Steuerkonsole (4), die wenigstens eine Betätigungsvorrichtung (17) und eine haptische Einrichtung (5, 15) umfasst, welche ihrerseits einen oder mehrere Positionierungssensoren und Servoaktuatoren umfasst, und wenigstens eine Robotereinheit (9, 23) mit:

einem Manipulatorarm (52),
einem Effektor (55), der am distalen Ende des Manipulatorarms (52) angeordnet ist, wobei der Effektor (55) mit wenigstens einem Kraft-Momenten-Sensor (10, 27) ausgestattet ist,
wenigstens einem Aktuator
und einem minimal invasiven Instrument (12, 13, 56), das mit dem Effektor (55) gekoppelt ist, wobei das distale Ende des minimal invasiven Instruments (12, 13, 56) so konfiguriert ist, dass es durch einen Drehpunkt (57) in eine Körperhöhle eines Patienten eingeführt wird,

wobei das Robotersystem eine Recheneinrichtung umfasst, die so konfiguriert ist, dass sie die folgenden Schritte ausführt:

Programmieren einer Position und Orientierung (39) des Effektors (55), basierend auf einer Relativbewegung (38) der haptischen Vorrichtung (15), auf Referenzkoordinaten (37) des Effektors (55), auf Koordinaten (45) des Effektors (55) gemäß einem Modell (25) der Robotereinheit (23) und auf einer Schätzung (48) der Position eines Drehpunktes;

basierend auf den Koordinaten (45) des Effektors (55) gemäß dem Modell (25) der Robotereinheit (23) und auf der programmierten Position und Orientierung (39) des Effektors (55): Erhalten von Gelenkgeschwindigkeiten und -positionen (42), die erforderlich sind, damit jeder Freiheitsgrad der Robotereinheit (23), die durch den Aktuator bewegt wird, es zusammen ermöglicht, die nächste programmierte Position und Orientierung (39) des Effektors (55) zu erreichen;

Bewegen des Effektors (55) mittels des wenigstens einen Aktuators entsprechend den Gelenkgeschwindigkeiten und -positionen (42);

Messen (46) von Kräften und Momenten, die durch den Effektor (55) und durch das damit gekoppelte minimal invasive Instrument (13, 56) ausgeübt werden, wenn diese Bewegung ausgeführt wird, mittels des wenigstens einen Kraft-Momenten-Sensors (10, 27), der mit dem Effektor (55) gekoppelt ist;

Bestimmen (28), welcher Prozentsatz der Messung (46) von Kräften und Momenten als Ergebnis der Wechselwirkung mit dem Drehpunkt (47) oder als Ergebnis der Wechselwirkung mit dem inneren Gewebe des Patienten (49) beigetragen wird, wobei diese Bestimmung basierend auf der Größe der Messung (46) durch Anwendung einer parametrisierten Sigmoidfunktion für jeden Beitrag durchgeführt wird;

erneutes Schätzen (48) der Position des Drehpunkts, wobei die Schätzung (48) basierend auf dem Beitrag, der sich aus der Wechselwirkung mit dem Drehpunkt (47) ergibt, und auf den Koordinaten (45) des Effektors (55) gemäß dem Modell (25) der Robotereinheit (23) durchgeführt wird;

Schätzen (50) der Steifigkeit des Gewebes in Kontakt mit dem distalen Ende des minimal invasiven Instruments (13, 56) und Berechnen einer simulierten Reaktionskraft (50);
Senden dieser simulierten Reaktionskraft (50) an den wenigstens einen Servoaktuator der haptischen Vorrichtung (15), um sie an die Hand des Chirurgen (14) zu übertragen.

2. System nach Anspruch 1, wobei die Koordinaten (45) des Effektors (55) gemäß dem Modell (25) der Robotereinheit (23) aus einer Gelenkposition (44) des Modells (25) erhalten werden.

3. System nach einem der vorhergehenden Ansprüche, wobei die Relativbewegung (38) der haptischen Vorrichtung (15) aus der Differenz zwischen einer absoluten Position und absoluten Verdrehung (32) der haptischen Vorrichtung (15) und einer Referenzposition und -verdrehung (18) erhalten wird.

4. System nach Anspruch 3, wobei auf die Differenz zwischen einer absoluten Position und absoluten Verdrehung (32) der haptischen Vorrichtung (15) und einer Referenzposition und -verdrehung (18) vom Chirurgen festgelegte Skalierungsfaktoren der Position $K_p$ und der Verdrehung $K_y$ zur Erhöhung der Bewegungspräzision und ein auf die simulierte Reaktionskraft (50) bezogener Reduktionsfaktor $K_s$ angewendet werden.

5. System nach einem der vorhergehenden Ansprüche, wobei zur Durchführung der Schätzung des Drehpunkts (48) die Recheneinrichtung ferner zur Schätzung des äußeren Abstands p entlang der Achse des minimal invasiven Instruments (13, 56) konfiguriert ist, in dem sich der Drehpunkt relativ zur Position (45) des Effektors (55) gemäß dem Modell (25) der Robotereinheit (23) befindet.

6. System nach einem der vorhergehenden Ansprüche, wobei die Schätzung (50) der Steifigkeit des Gewebes in Kontakt mit dem distalen Ende des minimal invasiven Instruments (13, 56) und die Berechnung einer simulierten Reaktionskraft (50) basierend auf dem Beitrag, der sich aus der Wechselwirkung mit dem inneren Gewebe des Patienten (49) ergibt, auf den Koordinaten (45) des Effektors (55) gemäß dem Modell (25) der Robotereinheit (23) und auf der Bewegung (51) der haptischen Vorrichtung (15), die von der Hand des Chirurgen (14) ausgeführt wird, durchgeführt werden.

7. System nach einem der vorhergehenden Ansprüche, wobei die Berechnung einer simulierten Reaktionskraft (50) basierend auf folgendem Ausdruck durchgeführt wird:

$$^{\{H\}}\mathbf{F_H} = -K_F K_T \left( {}^{\{H\}}\mathbf{P_H} - {}^{\{H\}}\mathbf{P_H^0} \right)$$

worin $^{\{H\}}\mathbf{F_H}$ für die simulierte Kraft (50) steht, $K_F$ ein Skalierungsfaktor ist, wobei $K_F < 1$ ist, $K_T$ die dynamische Steifigkeit ist, $^{\{H\}}\mathbf{P_H}$ für die aktuelle haptische Position (32) steht und $^{\{H\}}\mathbf{P_H^0}$ die letzte haptische Position ist.

8. System nach einem der vorhergehenden Ansprüche, wobei die Recheneinrichtung ferner dazu konfiguriert ist, basierend auf der Position und Orientierung (45) des Effektors (55) gemäß dem Modell (25) der Robotereinheit (23), auf der programmierten Position und Orientierung (39) und auf der Position und Orientierung (41) anderer Robotereinheiten (22), falls vorhanden, zu verifizieren, dass die programmierte Position und Orientierung für den Endeffektor (55) der Robotereinheit (9, 23) Sicherheitskriterien erfüllen, und die Position und Orientierung einzuschränken (40), falls sie die Kriterien nicht erfüllen.

9. System nach einem der vorhergehenden Ansprüche, wobei die Recheneinrichtung dazu konfiguriert ist, über eine Schnittstelle der Steuerkonsole (4) einen Bewegungstyp des minimal invasiven Instruments (13, 56) auszuwählen, d.h.: einen ersten Typ, bei dem die Bewegung der haptischen Vorrichtung (15) mit der Bewegung des distalen Endes des minimal invasiven Instruments (13, 56) zusammenhängt; oder einen zweiten Typ, bei dem die Bewegung der haptischen Vorrichtung (15) mit der Bewegung des Effektors (55) des Manipulatorarms zusammenhängt, so dass die Schwenkbewegungen eines manuellen minimal invasiven Instruments (13, 56) durch die haptische Vorrichtung (15) simuliert werden.

10. System nach einem der vorhergehenden Ansprüche, wobei die Recheneinrichtung so konfiguriert ist, dass sie einen der vorhergehenden Schritte ausführt, wenn die Betätigungseinrichtung (16) gedrückt oder betätigt wird.

11. System nach einem der vorhergehenden Ansprüche, wobei die Betätigungseinrichtung (16) ein Pedal ist.

12. Computersoftwareprodukt, das Anweisungen/Computerprogrammcode umfasst, um die Vorrichtung nach Anspruch 1 zu veranlassen, die folgenden Schritte auszuführen:

Programmieren einer Position und Orientierung (39) des Effektors (55), basierend auf einer Relativbewegung (38) der haptischen Vorrichtung (15), auf Referenzkoordinaten (37) des Effektors (55), auf Koordinaten (45) des Effektors (55) gemäß einem Modell (25) der Robotereinheit (23) und auf einer Schätzung (48) der Position eines Drehpunktes;

basierend auf den Koordinaten (45) des Effektors (55) gemäß dem Modell (25) der Robotereinheit (23) und auf der programmierten Position und Orientierung (39) des Effektors (55): Erhalten von Gelenkgeschwindigkeiten und -positionen (42), die erforderlich sind, damit jeder Freiheitsgrad der Robotereinheit (23), die durch den Aktuator bewegt wird, es zusammen ermöglicht, die nächste programmierte Position und Orientierung (39) des Effektors (55) zu erreichen;

Bewegen des Effektors (55) mittels des wenigstens einen Aktuators entsprechend den Gelenkgeschwindigkeiten und -positionen (42);

Messen (46) von Kräften und Momenten, die durch den Effektor (55) und durch das damit gekoppelte minimal invasive Instrument (13, 56) ausgeübt werden, wenn diese Bewegung ausgeführt wird, mittels des wenigstens einen Kraft-Momenten-Sensors (10, 27), der mit dem Effektor (55) gekoppelt ist;

Bestimmen (28), welcher Prozentsatz der Messung (46) von Kräften und Momenten als Ergebnis der Wechselwirkung mit dem Drehpunkt (47) oder als Ergebnis der Wechselwirkung mit dem inneren Gewebe des Patienten (49) beigetragen wird, wobei diese Bestimmung basierend auf der Größe der Messung (46) durch Anwendung einer parametrisierten Sigmoidfunktion für jeden Beitrag durchgeführt wird;

erneutes Schätzen (48) der Position des Drehpunkts, wobei die Schätzung (48) basierend auf dem Beitrag, der sich aus der Wechselwirkung mit dem Drehpunkt (47) ergibt, und auf den Koordinaten (45) des Effektors (55) gemäß dem Modell (25) der Robotereinheit (23) durchgeführt wird;

Schätzen (50) der Steifigkeit des Gewebes in Kontakt mit dem distalen Ende des minimal invasiven Instruments (13, 56) und Berechnen einer simulierten Reaktionskraft (50);

Senden dieser simulierten Reaktionskraft (50) an den wenigstens einen Servoaktuator der haptischen Vorrichtung (15), um sie an die Hand des Chirurgen (14) zu übertragen.

13. Computerlesbares Medium, auf dem das Computerprogramm nach Anspruch 12 gespeichert ist.

## Revendications

1. Système robotique pour la chirurgie mini-invasive, dans lequel le système robotique comprend :

une console de commande (4) comprenant au moins un dispositif d'actionnement (17) et un dispositif haptique (5, 15) qui à son tour comprend un ou plusieurs capteurs de position et des servomoteurs, et au moins une unité robotique (9, 23) comprenant :

un bras manipulateur (52),
un effecteur (55) disposé à l'extrémité distale du bras manipulateur (52), lequel effecteur (55) est équipé d'au moins un capteur de force et de moment (10, 27),
au moins un actionneur,
et un instrument mini-invasif (12, 13, 56) couplé à l'effecteur (55), l'extrémité distale de l'instrument mini-invasif (12, 13, 56) étant configurée pour être introduite dans une cavité du corps d'un patient par un point fixe (57),

le système robotique comprenant des moyens de calcul configurés pour effectuer les étapes suivantes :

programmer une position et une orientation (39) de l'effecteur (55) sur la base d'un déplacement relatif (38) du dispositif haptique (15), de coordonnées de référence (37) de l'effecteur (55), de coordonnées (45) de l'effecteur (55) selon un modèle (25) de l'unité robotique (23), et d'une estimation (48) de la position d'un point fixe ;
sur la base des coordonnées (45) de l'effecteur (55) selon le modèle (25) de l'unité robotique (23) et de la position et l'orientation programmées (39) de l'effecteur (55), obtenir des vitesses et des positions articulaires (42) requises pour que chaque degré de liberté de l'unité robotique (23) déplacée par l'actionneur, ensemble, permette d'atteindre la position et l'orientation programmées suivantes (39) de l'effecteur (55) ;

déplacer l'effecteur (55) au moyen dudit au moins un actionneur en fonction des vitesses et positions articulaires (42) ;

mesurer (46), au moyen dudit au moins un capteur de force et de moment (10, 27) couplé à l'effecteur (55), les forces et les moments exercés par l'effecteur (55) et par l'instrument mini-invasif (13, 56) couplé à celui-ci lorsque ce mouvement est effectué ;

déterminer (28) quel pourcentage de la mesure (46) des forces et des moments est contribué en raison de l'interaction avec le point fixe (47) ou en raison de l'interaction avec le tissu interne du patient (49), cette détermination étant effectuée sur la base de l'amplitude de la mesure (46) en appliquant une fonction sigmoïde paramétrée pour chaque contribution ;

estimer (48) à nouveau la position du point fixe, l'estimation (48) étant effectuée sur la base de la contribution résultant de ladite interaction avec le point fixe (47) et des coordonnées (45) de l'effecteur (55) selon le modèle (25) de l'unité robotique (23) ;

estimer (50) la rigidité du tissu en contact avec l'extrémité distale de l'instrument mini-invasif (13, 56) et calculer une force de réaction simulée (50) ;

envoyer cette force de réaction simulée (50) à l'au moins un servomoteur du dispositif haptique (15) pour qu'elle soit fournie à la main du chirurgien (14).

2. Système selon la revendication 1, dans lequel les coordonnées (45) de l'effecteur (55) selon le modèle (25) de l'unité robotique (23) sont obtenues à partir d'une position articulaire (44) du modèle (25).

3. Système selon l'une quelconque des revendications précédentes, dans lequel le déplacement relatif (38) du dispositif haptique (15) est obtenu à partir de la différence entre une position absolue et une torsion absolue (32) du dispositif haptique (15) et une position et une torsion de référence (18).

4. Système selon la revendication 3, dans lequel des facteurs d'échelle de position $K_p$ et de torsion $K_\gamma$ établis par le chirurgien pour augmenter la précision du mouvement, et un facteur de réduction $K_s$ lié à la force de réaction simulée (50), sont appliqués à la différence entre une position absolue et une torsion absolue (32) du dispositif haptique (15) et une position et une torsion de référence (18).

5. Système selon l'une quelconque des revendications précédentes, dans lequel pour effectuer l'estimation du point fixe (48), les moyens de calcul sont en outre configurés pour estimer la distance externe $p$. le long de l'axe de l'instrument mini-invasif (13, 56), à laquelle est située le point fixe par rapport à la position (45) de l'effecteur (55) selon le modèle (25) de l'unité robotique (23).

6. Système selon l'une quelconque des revendications précédentes, dans lequel l'estimation (50) de la rigidité du tissu en contact avec l'extrémité distale de l'instrument mini-invasif (13, 56) et le calcul d'une force de réaction simulée (50) sont effectués sur la base de la contribution résultant de l'interaction avec le tissu interne du patient (49), des coordonnées (45) de l'effecteur (55) selon le modèle (25) de l'unité robotique (23), et du déplacement (51) du dispositif haptique (15) effectué par la main du chirurgien (14).

7. Système selon l'une quelconque des revendications précédentes, dans lequel le calcul d'une force de réaction simulée (50) est effectué sur la base de l'expression suivante :

$$^{\{H\}}\mathbf{F}_{\mathbf{H}} = -K_F K_T \left( {}^{\{H\}}\mathbf{P}_{\mathbf{H}} - {}^{\{H\}}\mathbf{P}_{\mathbf{H}}^{0} \right)$$

où $^{\{H\}}F_H$ représente la force simulée (50), $K_F$ est un facteur d'échelle, avec $K_F < 1$, $K_T$ est la rigidité dynamique, $^{\{H\}}\mathbf{P_H}$ est la position haptique actuelle (32) et $^{\{H\}}\mathbf{P_H^0}$ est la dernière position haptique.

8. Système selon l'une quelconque des revendications précédentes, dans lequel les moyens de calcul sont en outre configurés pour : sur la base de la position et de l'orientation (45) de l'effecteur (55) selon le modèle (25) de l'unité robotique (25), de la position et de l'orientation programmées (39) et de la position et de l'orientation (41) d'autres unités robotiques (22), s'il y en a, vérifier que la position et l'orientation programmées pour l'effecteur final (55) de l'unité robotique (9, 23) sont conformes à des critères de sécurité, et restreindre (40) les position et orientation si elles ne sont pas conformes aux critères.

9. Système selon l'une des revendications précédentes, dans lequel les moyens de calcul sont configurés pour : par le biais d'une interface de la console de commande (4), sélectionner un type de mouvement de l'instrument mini-invasif (13, 56) : un premier type dans lequel le déplacement du dispositif haptique (15) est lié au déplacement de l'extrémité distale de l'instrument mini-invasif (13, 56) ; ou un deuxième type dans lequel le déplacement du dispositif haptique (15) est lié au déplacement de l'effecteur (55) du bras manipulateur, de sorte que les mouvements de pivotement d'un instrument manuel mini-invasif (13, 56) sont simulés par le biais du dispositif haptique (15).

10. Système selon l'une quelconque des revendications précédentes, dans lequel les moyens de calcul sont configurés pour effectuer l'une quelconque des étapes précédentes si ledit dispositif d'actionnement (16) est poussé ou actionné.

11. Système selon l'une quelconque des revendications précédentes, dans lequel le dispositif d'actionnement (16) est une pédale.

12. Produit logiciel informatique comprenant des instructions/un code de programme informatique pour amener le dispositif de la revendication 1 à exécuter les étapes suivantes :

programmer une position et une orientation (39) de l'effecteur (55) sur la base d'un déplacement relatif (38) du dispositif haptique (15), de coordonnées de référence (37) de l'effecteur (55), de coordonnées (45) de l'effecteur (55) selon un modèle (25) de l'unité robotique (23), et d'une estimation (48) de la position d'un point fixe ;
sur la base des coordonnées (45) de l'effecteur (55) selon le modèle (25) de l'unité robotique (23) et de la position et l'orientation programmées (39) de l'effecteur (55), obtenir des vitesses et des positions articulaires (42) requises pour que chaque degré de liberté de l'unité robotique (23) déplacée par l'actionneur, ensemble, permette d'atteindre la position et l'orientation programmées suivantes (39) de l'effecteur (55) ;
déplacer l'effecteur (55) au moyen dudit au moins un actionneur en fonction des vitesses et positions articulaires (42) ;
mesurer (46), au moyen dudit au moins un capteur de force et de moment (10, 27) couplé à l'effecteur (55), les forces et les moments exercés par l'effecteur (55) et par l'instrument mini-invasif (13, 56) couplé à celui-ci lorsque ce mouvement est effectué ;
déterminer (28) quel pourcentage de la mesure (46) des forces et des moments est contribué en raison de l'interaction avec le point fixe (47) ou en raison de l'interaction avec le tissu interne du patient (49), cette détermination étant effectuée sur la base de l'amplitude de la mesure (46) en appliquant une fonction sigmoïde paramétrée pour chaque contribution ;
estimer (48) à nouveau la position du point fixe, l'estimation (48) étant effectuée sur la base de la contribution résultant de l'interaction avec le point fixe (47) et des coordonnées (45) de l'effecteur (55) selon le modèle (25) de l'unité robotique (23) ;
estimer (50) la rigidité du tissu en contact avec l'extrémité distale de l'instrument mini-invasif (13, 56) et calculer une force de réaction simulée (50) ;
envoyer cette force de réaction simulée (50) à l'au moins un servomoteur du dispositif haptique (15) pour qu'elle soit fournie à la main du chirurgien (14).

13. Support lisible par ordinateur sur lequel est stocké le programme informatique de la revendication 12.

FIG. 1

FIG. 2

FIG. 3

FIG. 4

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- ES 2298051 B2
- US 20070276423 A1
- US 7025064 B2
- WO 2011125007 A1
- US 2013012930 A1
- US 2014005682 A1
- US 20130012930 A1
- US 2015359597 A1
- WO 2015158756 A
- WO 2016064632 A